# EUROPÄISCHE PATENTSCHRIFT

(11) **EP 0 273 004 B1**
(45) Veröffentlichungstag und Bekanntmachung des Hinweises auf die Patenterteilung: **11.08.1993**
(21) Anmeldenummer: 87810667.3
(22) Anmeldetag: 16.11.1987
(51) Int. Cl.: A61M 35/00

(54) **Benutzer-aktivierte Therapeutische Systeme**
User-activated therapeutical system
Système thérapeutique activé par l'utilisateur

(30) Priorität: 20.11.1986 US 933000; 13.02.1987 US 14313
(43) Veröffentlichungstag der Anmeldung: 29.06.1988
(73) Patentinhaber: CIBA-GEIGY AG, 4002 Basel (CH)
(72) Erfinder: Heiber, Werner E., Bedford Hills New York 10507 (US); Andriola, Robert, Putnam Valley New York 10579 (US); Williams, Paul, Fairlawn New Jersey 07410 (US); Ebert, Charles, Redwood City California 94063 (US)

(56) Entgegenhaltungen:
- DE-A- 3 439 239
- US-A- 3 053 255
- US-A- 3 598 122
- US-A- 3 598 123
- US-A- 3 731 683
- US-A- 3 734 097
- US-A- 3 742 951
- US-A- 3 797 494
- US-A- 4 476 976
- US-A- 4 597 961

## Beschreibung

Gegenstand der Erfindung ist ein therapeutisches Pflaster für die transdermale oder topische Anwendung bestehend aus einem Hohlraum gefüllt mit Wirkstoff und einem Weiteren Hohlraum gefüllt mit einem Aktivierungsmittel, wobei der Wirkstoff durch Einwirkung des Aktivierungsmittels hautpermeabel wird.

Seit einiger Zeit haben Pflaster zur Verabreichung von Wirkstoffen mittels Absorption durch die Haut (Transdermale Therapeutische Systeme - TTS) Bedeutung erlangt. Der Vorteil dieser Darreichungsform besteht im Übergang von Wirkstoffen in die Blutgefässe unter Umgehung des gastrointestinalen Trakts. Der Durchgang durch das hepatische System wird vermieden, bevor der Wirkstoff an die spezifischen Rezeptoren gelangt. Ausserdem können mittels transdermaler Verabreichung Wirkstoffe im Wege der Selbstmedikation durch den Patienten selbst verabreicht werden.

In den bekannten Transdermalen Therapeutischen Systemen, z.B. Nitroderm TTS® (Ciba-Geigy), kann die Wirkstoffkomponente die zwischen dem Wirkstoffreservoir und der Anwendungsfläche gelegene Membranschicht durchdringen und sich in der Klebschicht anreichem, was eine unerwünscht hohe Initialdosis bewirken kann. Einige Wirkstoffe besitzen in TTS ausserdem eine niedrige Lagerstabilität. Wegen des hohen Dampfdrucks sind bestimmte Wirkstoffe, z.B. Nicotin oder Arecolin, bei der Verarbeitung problematisch.

Der vorliegenden Erfindung liegt die Aufgabe zugrunde therapeutische Pflaster mit verbesserter Lagerstabilität herzustellen, welche sich darüberhinaus durch kontinuierliche und kontrollierte Abgabe des Wirkstoffs auszeichnen.

In der US-A-3 053 255 sind therapeutische Pflaster beschrieben, welche aus übereinander liegenden Hohlräumen bestehen, die jeweils mit Wirkstoff und flüssigen Formulierungsbestandteilen gefüllt sind. Die Verbindung zwischen den beiden Hohlräumen erfolgt mit Verbindungssträngen, welche als Dochte geformt sind und den Transport von Flüssigkeiten ermöglichen.

Solche Pflaster sind nachteilig, da nur geringe Flüssigkeitsmengen zwischen den Hohlräumen ausgetauscht werden können. Bis zum Einsetzen der Abgabe einer für den therapeutischen Effekt ausreichenden hautpermeablen Wirkstoffmenge wird erst ein längerer Zeitraum verstreichen.

Die Nachteile dieses therapeutischen Pflasters werden durch die vorliegende Erfindung überwunden, welche ein Pflaster mit nebeneinander liegenden Hohlräumen betrifft. Durch Aufbrechen der dazwischenliegenden Trennschicht können die in den Hohlräumen befindlichen Formulierungsbestandteile in kurzer Zeit vermischt werden, so dass zum gewünschten Zeitpunkt durch Einwirken eines vom Wirkstoff räumlich getrennten Aktivierungsmittels auf den zu applizierenden Wirkstoff dieser hautpermeabel wird.

Die vorliegende Erfindung für alle Vertragsstaaten außer Spanien u. Griechenland betrifft ein therapeutisches Pflaster für die transdermale oder topische Anwendung, bestehend aus einem Hohlraum gefüllt mit einem Wirkstoff und einem weiteren Hohlraum gefüllt mit einem Aktivierungsmittel, wobei beide Hohlräume durch eine für Aktivierungsmittel und Wirkstoff undurchlässige Schicht getrennt sind und anwendungsseitig von einer Membran mit einer Klebschicht sowie einer darauf abziehbar aufgebrachten Schutzschicht und auf der Seite, welche der Anwendungsseite abgewandt ist, von einer Deckschicht begrenzt sind, wobei letztere geschlossen und gegenüber dem Wirkstoff und/oder dem Aktivierungsmittel undurchlässig ist, dadurch gekennzeichnet, dass die gefüllten Hohlräume nebeneinander liegen, die Trennschicht berstfähig ist und nach dem Bersten der Trennschicht infolge Druckeinwirkung des Benutzers der Wirkstoff durch Einwirkung des Aktivierungsmittels hautpermeabel wird.

Die berstfähige Trennschicht wird durch Druckeinwirkung des Benutzers zum Bersten gebracht. Dies kann kurz vor, während oder kurz nach dem Anbringen des Systems auf der Haut geschehen. Dabei tritt das ursprünglich in einem Hohlraum des Systems befindliche Aktivierungsmittel mit der inaktiven Wirkstoffkomponente aus dem anderen Hohlraum in Wechselwirkung, wobei die Wirkstoffkomponente aktiviert wird. Durch chemische Reaktion des Aktivierungsmittels mit dem Wirkstoff wird die Geschwindigkeit der Wirkstoffabgabe vom System auf die Haut in Gang gesetzt oder beschleunigt.

Ein anderer Aspect der Erfindung beinhaltet ein Verfahren zur Herstellung eines Therapeutischen Pflasters.

### Zeichnungen:

Figur 1 zeigt eine erfindungsgemässe Ausführungsform (1)
mit nebeneinander liegenden Hohlräumen mit Blick von oben.

Figur 2 ist eine Ansicht der Ausführungsform (1) gemäss Figur 1 entlang der Linie **2-2**.

### Erklärung der Bezugszeichen:

Die anwendungsseitig (der Haut zugewandt) am nächsten liegende Schicht 8 ist eine abziehbar aufgebrachte Schutzschicht, welche vom Benutzer oder einer anderen Person vor dem Anbringen des Pflasters auf die Haut entfernt wird. Das applikationsfähige Pflaster mit den Bestandteilen 2-7 und 9 (8 ist entfernt) wird als Ganzes auf der Haut des Patienten befestigt, wobei die Klebschicht 7 mit der Haut verbunden ist. Die Klebschicht 7 ist ebenfalls mit der Membran 6 verbunden, welche die Abgabegeschwindigkeit des Wirkstoffs kontrolliert. Die Deckschicht 5 ist geschlossen (undurchlässis) und mit der Membran 6 verbunden oder vorzugsweise verschweisst, so dass beide die Hohlräume 2 und 3 bilden. Die beiden Hohlräume sind durch die in Form eines Steges gebildete berstfähige Trennschicht 4 voneinander getrennt.

Einer der beiden Hohlräume 2 und 3 enthält als Reservoir das Aktivierungsmittel, während der andere eine inaktive oder zu aktivierende Form des Wirkstoffs enthält.

Die untere Lasche 9' ist ein Bestandteil der abziehbaren Schutzschicht 8, welche nicht mit Klebstoff beschichtet ist und vom Pflaster 1 absteht, so dass sich die abziehbare Schutzschicht 8 leicht entfernen lässt. Die obere Lasche 9 ist ebenfalls nicht mit Klebstoff beschichtet und dient zur Entfernung des Pflasters von der Haut des Patienten. 11 stellt eine umlaufende Siegelnaht dar.

Ebenfalls Gegenstand der Erfindung ist ein Verfahren zur Herstellung des therapeutischen Pflasters (1), welches dadurch gekennzeichnet ist, dass man auf der abziehbaren Schutzschicht (8) die Klebschicht (7) und darauf die Membran (6) aufträgt, auf die Membran den Inhalt der beiden Hohlräume (2,3) mit dem Wirkstoff und dem Aktivierungsmittel nebeneinander bringt, über den Inhalt der beiden Hohlräume die Deckschicht (5) legt und diese Deckschicht mit der Membran (6) verschweisst und eine undurchlässige Trennschicht (4) bildet, welche durch Druckeinwirkung des Benutzers berstfähig ist, so dass der Wirkstoff durch Einwirkung des Aktivierungsmittels hautpermeabel wird.

Siliconisierter Polyester, ca. 75 Mikrometer stark, kann als abziehbare Schutzschicht 8 verwendet werden. Auf diese Schicht wird die Klebschicht 7, z.B. eine Polyisobutylen-Lösung, aufgetragen. Auf diese Klebschicht, z.B. aus Aethylen-Vinylacetat-Copolymerisat (EVA), wird die Membran 6 mit ca. 100 Mikrometer Stärke aufgetragen. Auf die Membran kann der Inhalt der Hohlräume 2 und 3 nebeneinander gebracht werden. Die Deckschicht 5 mit ca. 80 Mikrometer Stärke wird über den Inhalt der Hohlräume 2 und 3 so gelegt, dass eine verschweissfähige, z.B. durch Wärme, Beschichtung dem Inhalt der Hohlräume zugewandt ist. Man stellt durch Verschweissen die Siegelnaht 11 dem Umfang der Darreichungsform entsprechend her und bringt ebenfalls durch Verschweissen die Siegelnaht 4 zwischen den Hohlräumen 2 und 3 an. Die Stärke der Siegelnaht 4 wird so gewählt vorzugsweise 0,5 bis 2 mm, dass nur diese durch Druckwirkung des Benutzers (vorzugsweise Druckwirkung des Benutzers (vorzugsweise 4 bis 20kg) zum Bersten gebracht wird, wobei die Siegelnaht 11 unversehrt bleibt. Die erforderliche Kraft zum Bersten der Siegelnaht 4 sollte mindestens bei 4 kg liegen.

Durch Druckeinwirkung darf nur die Siegelnaht 4 zum Bersten gebracht werden. Zweckmässigerweise wird die nicht-berstfähige Siegelnaht 11 so gestaltet, dass diese im Vergleich mit der berstfähigen Siegelnaht 4 zumindest den 1,5-fachen, vorzugsweise den 2-fachen, oder den 2,5-fachen, Druck aushalten muss. Selbstverständlich müssen bei der Anwendung von Druck alle anderen Teile des therapeutischen Systems, insbesondere die Membran 6, mit Ausnahme der Siegelnaht 4 erhalten bleiben.

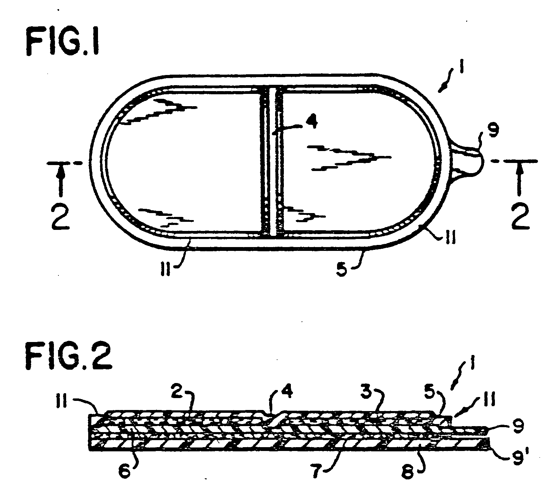

Die Ausführungsform gemäss Figur 1 kann beliebige Formgebung haben. Die längliche Formgebung gemäss Figur 1 oder kreisrunde Formen sind bevorzugt.

Die berstfähige Trennschicht hat eine Stärke von ca. 20 - 80 %, insbesondere von ca. 20 - 50 %, der Stärke der anderen Membranen aus demselben Material im System.

Der Benutzer bringt die Trennschicht 4 vor dem Anbringen des Pflasters auf der Haut oder vorzugsweise kurz danach zum Bersten. Dadurch kann der Inhalt der Hohlräume 2 und 3 miteinander in Wechselwirkung treten und das therapeutische System aktiviert werden. Umfasst vom Gegenstand der Erfindung sind sämtliche Systeme, in denen nach diesem Aufbrechen eine Aktivierung stattfindet, sei es durch Veränderung der Permeabilität des Wirkstoffs durch Einwirken des aktivierenden Mittels, z.B. durch Einwirkung eines Permeationsverbesserers, oder durch Ueberführen des Wirkstoffs von einer nicht permeationsfähigen in eine permeationsfähige Form, z.B. durch chemische Reaktion, z.B. durch Umwandlung eines nicht permeationsfähigen Salzes in eine permeationsfähige Säure oder Base durch Säure-Basen-Reaktion mit einem aktivierenden Mittel, das seinerseits eine stärkere Säure oder Base darstellt.

Alle Ausführungsformen sind Gegenstand der vorliegenden Erfindung, worin nach Aufbrechen der Trennschicht das aktivierende Mittel mit einer inaktiven oder weniger aktiven Form des Wirkstoffs in Wechselwirkung tritt und das System als Ganzes in seine aktive Form überführt wird.

Bei Verwendung einer durch Einwirkung von Wärme verschweissten Siegelnaht 4 als Trennschicht zwischen den Reservoirkammern (Hohlräumen) sollte diese Siegelnaht weniger stark als die umlaufende Siegelnaht sein, welche das System verschliesst. So ist gewährleistet, dass nur die dünnere Trennschicht zum Bersten gebracht wird. Um unbeabsichtigtes Aufbrechen der Trennschicht zu verhindern, sollte diese so stark verschweisst sein, dass sie Kräften von 3 bis 7 kg widersteht, insbesondere Kräften von 5 - 7 kg. Bei Anwendung von höheren Kräften kann diese dann gezielt zum Bersten gebracht werden.

Inaktive Formen des Wirkstoffs oder Vorstufen davon sind beispielsweise Pulver, Kristalle, ionisierte Formen, z.B. gebunden an Ionenaustauscher, Wirkstoffe gebunden mit schwacher Bindung an immobile Phasen, Wirkstoffe in verkapselter Form, in Form einer chemischen Vorstufe oder als Prodrug. Alle denkbaren weniger aktiven Vorstufen sind umfasst.

In einer bevorzugten Ausführungsform kann die inaktive Form des Wirkstoffs von der Haut nicht aufgenommen werden und/oder mindestens eine Trennschicht zwischen dem therapeutischen System und der Haut (oder der abziehbaren Schutzschicht 8) nicht durchdringen. Dies lässt sich anhand der Applikation eines Wirkstoffs mit sauren oder basischen Eigenschaften illustrieren, der in ionischer und/oder dissoziierter Form kaum permeationsfähig ist, aber als freie Säure oder Base Membranen und Klebschicht durchdringt und von der Haut aufgenommen wird. Bei Anbringen des Pflasters und vor der Aktivierung des Systems wird nur eine geringe Dosis abgegeben. Nach der Aktivierung werden therapeutische Dosen aufgenommen.

Als aktivierende Mittel eignen sich alle pharmazeutisch unbedenklichen Stoffe, welche den Wirkstoff in seiner inaktiven oder weniger aktiven Form in eine aktive Form umwandeln, so dass eine therapeutische wirksame Dosis in der gewünschten Geschwindigkeit dem Patienten topisch verabreicht wird. Solche aktivierenden Mittel sind beispielsweise Lösungsmittel wie Wasser, Alkohol, pH-Regulatoren wie Pufferlösungen, Säuren und Basen, welche die entsprechenden basischen und sauren Wirkstoffe als freie Säuren oder Basen freisetzen und als solche permeationsfähig sind, Salzlösungen, welche den Wirkstoff eluieren, Enzyme oder Katalysatoren, welche labile Bindungen lockern, quell- oder spreitungsfähige Mittel, welche mikroverkapselte Poren öffnen, reaktive Spezies, welche den Wirkstoff aus seiner Vorstufe freisetzen oder als Prodrug in die wirksame Form überführen etc..

Für die erfindungsgemässen therapeutischen Systeme können beliebige Wirkstoffe verwendet werden. Insbesondere können systemische Wirkstoffe in der vorgesehenen Dosierung für die bekannten therapeutischen Verfahren verwendet werden. Geeignete systemische Wirkstoffe sind unter anderem Antibiotika aus der Gruppe der Penicilline, Tetracyclin, Oxytetracylin, Chlortetracylin, Chloramphenicol, Cephalosporine, Peneme, Aminoglycoside und Sulfonamide, Sedativa wie Pentabarbital-Natrium, Codein, Bromisovalerylharnstoff, Carbromal und Natriumphenobarbital, Stimulanzien mit Wirkung auf das zentrale Nervensystem wie 3-(2-Aminopropyl)indolacetat oder 3-(2-Aminobutyl)indolacetat, Beruhigungsmittel wie Reserpin, Chlorpromazinhydrochlorid oder Thiopropazathydrochlorid, Hormone vom Adrenocorticosteroid-Typ wie Methylpredhisolon, androgene Steroide wie Methyltestosteron oder Fluoxymesteron, oestrogene Steroide wie Oestron, 17β-Oestradiol oder Aethinylöstradiol, progestagene Steroide wie 17α-Acetoxyprogesteron, Medroxyprogesteronacetat, 19-Norprogesteron oder Norethindron, Schilddrüsenhormone wie Thyroxin, Antipyretika wie Aspirin, Salicylamid oder Natriumsalicylat, Morphin und andere Analgetika auf Morphinbasis, Antidiabetika wie Insulin, Herz-Kreislaufmittel wie Nitroglycerin, Betablocker wie Metoprolol oder Oxprenolol, Digitalispräparate wie Digitoxin, Digoxin oder Ouabain, Anticholinergika wie Atropin, Scopolamin-hydrobromid, Methscopolaminbromid oder Hyoscyamin, Antimalariamittel aus der Gruppe der 4-Aminochinoline, 9-Aminochinoline sowie Pyrimethamin, Nahrungsmittelzusätze wie Vitamine, essentielle Aminosäuren oder essentielle Fette.

Statt der genannten systemischen Wirkstoffe können auch topische Wirkstoffe in der vorgesehenen Dosierung für die bekannten therapeutischen Verfahren verwendet werden.

Geeignete topische Wirkstoffe sind beispielsweise Antiperspiranzien wie Aluminiumchlorid, Deodoranzien wie Triclosan oder Methylbenzethoniumchlorid, adstringierende Mittel wie Tanninsäure, Irritanzien wie Methylsalicylat oder Kampher, Kantharidine, Keratolytika wie Benzoesäure, Salicylsäure, Resorcin, Iodochlorhydroxyquin, antifungische Mittel wie Tolnaftat, Griseofulvin, Mystatin oder Amphotericin, Antiinflammatorika von Corticosteroidtyp, z.B. Hydrocortison, Hydrocortisonacetat, Prednisolon, Methylprednisolon, Triamcinolonacetonid, Fludrocortison, Flurandrenolon, Flumethason, Dexamethason, Betamethason, Fluocinolonacetonid, Fluorometholon oder Pramoxin, Antineoplastika, z.B. Methotrexat, antibakterielle Mittel wie Bacitracin, Neomycin, Erythiomycin, Tetracyclin, Chlortetracyclin, Chloramphenicol, Oxytetracyclin, Polymycin B, Nitrofuraxon, Mafenid, Benzalkoniumchlorid, Cetalkoniumchlorid, Methylbenzethoniumchlorid oder Neomycinsulfat.

Zu den besonders bevorzugten Wirkstoffen gehören: Fentanyl, Terbutalin, Formoterol, Theophyllin, 17β-Oestradiol, Progesteron, Scopolamin, Nitroglycerin, Triprolidin, Tripelenamin, Diphenylhydramin, Arecolin, Nicotin oder 3-[(5-Amino-1-carboxy)-pentylamino]-1-carboxymethyl-2,3,4,5-tetrahydro-1H-1-benzazepine-2-one. Von den genannten Wirkstoffen sind vor allem Nitroglycerin, Scopolamin, 17β-Oestradiol und Nicotin besonders bevorzugt.

Die folgenden Beispiele sollen die Erfindung illustrieren, ohne deren Umfang zu beschränken.

### Beispiel 1: Membransystem

Dieses Beispiel illustriert eine Ausführungsform gemäss den Figuren 1 und 2. Das transdermale thereapeutische System hat eine Abgabefläche für den Wirkstoff von ca. 10 cm² und wird folgendermassen hergestellt:
Eine Konaktkleberlösung aus Polyisobutylen in Hexan wird auf eine 75 Mikrometer starke Schicht aus silikonisiertem Polyester gegossen. Nach dem Trocknen wird die mit Kleber beschichtete Seite auf ein 100 Mikrometer starke Membranschicht aus Aethylen-Vinylacetat-Copolymerisat (EVA) beschichtet, wobei auf einer Seite ein Streifen frei von Klebstoff bleibt. Der Inhalt der Wirkstoffkammer und der Kammer mit dem Aktivierungsmittel werden nebeneinander auf die EVA-Schicht des Laminats gleichzeitig aufgetragen. Die Wirkstoffkammer enthält 183,6 mg einer salbenförmigen Formulierung mit 58,5 % Arecolinhydrobromid, 1,7 % CARBOPOL 934 P Quellmittel und 39,8% Wasser. Der Gehalt an Arecolinhydrobromid in dieser Formulierung beträgt 107,4 mg oder 0,455 mMol. Die Kammer mit dem Aktivierungsmittel enthält 121,3 mg einer gelförmigen Formulierung mit 51,88 % Kaliumcarbonat, 1,57 % CARBOPOL 934 P Quellmittel und 39,8 % Wasser. Der Gehalt an Kaliumcarbonatbase in dieser Formulierung beträgt 62,39 mg oder 0,455 mMol.

Eine 80 Mikrometer starke undurchlässige Deckschicht aus Polyester mit einer heiss verschweissbaren EVA-Beschichtung wird über die Formulierungsmassen gelegt, am Rande heiss verschweisst und gleichzeitig oder anschliessend der Inhalt der beiden Kammern durch Verschweissen einer Siegelnaht zwischen den Kammern räumlich voneinander getrennt. Die Siegelnaht zwischen den Kammern ist schwächer als die umlaufende Siegelnaht ausgeprägt, so dass die Siegelnaht bereits bei einem Druck von ca. 3 kg/cm² bersten kann.

Das System wird vom Benutzer durch Bersten der Siegelnaht und durch Vermischen der in den Kammern befindlichen Komponenten aktiviert. Dabei wird durch Einwirken der Base das nicht permeationsfähige Wirkstoffsalz in die permeationsfähige freie Wirkstoffbase umgewandelt. In-vitro lässt sich zeigen, dass auf einer Abgabefläche von ca. 10 cm² 22,5 mg freie Base innerhalb 24 Stunden abgegeben werden.

### Beispiel 2: Membransystem

Dieses Beispiel illustriert eine Ausführungsform gemäss den Figuren 1 und 2. Das transdermale therapeutische System hat eine Abgabefläche von ca. 10 cm² und wird folgendermassen hergestellt:
Ein Silikonkontaktkleber wird auf eine ca. 75 Mikrometer starke Schicht aus Fluorkohlenwasserstoffpolyester warm aufgeschmolzen. Die mit Klebstoff versehene Seite wird auf eine ca. 75 Mikrometer starke EVA-Membranschicht beschichtet. Darauf wird der Inhalt der Wirkstoffkammer und der Kammer mit dem Aktivierungsmittel nebeneinander gleichzeitig aufgetragen. Die Wirkstoffkammer enthält 157,7 mg einer gelförmigen Formulierung von Nicotindihydrochlorid bestehend aus 44,9 % Nicotindihydrochlorid, 3,8 % CARBOPOL 934 P und 48,7 % Wasser. 157,7 mg der Formulierung enthalten 70,8 mg oder 0,301 mMol Nicotindihydrochlorid. Die Kammer mit dem Aktivierungsmittel enthält 226 mg einer gelförmigen Formulierung mit 14,2 % Natriumhydroxid, 2,7 % CARBOPOL 934 P und 83,1 % Wasser. Der Gehalt an Natriumhydroxid in dieser Formulierung beträgt 32,1 mg oder 0,802 mMol Natriumhydroxid.

Eine 80 Mikrometer starke undurchlässige Deckschicht aus Polyester mit einer heiss verschweissbaren EVA-Beschichtung wird über die Formulierungsmassen gelegt, am Rande heiss verschweisst und gleichzeitig oder anschliessend der Inhalt der beiden Kammern durch Verschweissen einer Siegelnaht zwischen den Kammern räumlich voneinander getrennt. Die Siegelnaht zwischen den Kammern ist schwächer als die umlaufende Siegelnaht ausgeprägt, so dass die Siegelnaht bei einem Druck von ca. 3 kg/cm² bersten kann.

Das System wird vom Benutzer durch Bersten der Siegelnaht und durch Vermischen der in den Kammern befindlichen Komponenten aktiviert. Dabei wird durch Einwirken der Base das nicht permeationsfähige Wirkstoff-Salz in die permeationsfähige freie Wirkstoff-Base umgewandelt. In-vitro lässt sich zeigen, dass auf einer Abgabefläche von ca. 10 cm² 16,5 mg freie Base innerhalb 24 Stunden abgegeben werden.

## Patentansprüche

1. Therapeutisches Pflaster (1) für die transdermale oder topische Anwendung bestehend aus einem Hohlraum (2,3) gefüllt mit einem Wirkstoff und einem weiteren Hohlraum (2,3) gefüllt mit einem Aktivierungsmittel, wobei beide Hohlräume durch eine für Aktivierungsmittel und Wirkstoff undurchlässige Schicht (4) getrennt sind und anwendungsseitig von einer Membran (6) mit einer Klebschicht (7) sowie einer darauf abziehbar aufgebrachten Schutzschicht (8) und auf der Seite, welche der Anwendungsseite abgewandt ist, von einer Deckschicht (5) begrenzt sind, wobei letztere geschlossen und gegenüber dem Wirkstoff und/oder dem Aktivierungsmittel undurchlässig ist, dadurch gekennzeichnet, dass die gefüllten Hohlräume (2,3) nebeneinander liegen, die Trennschicht (4) berstfähig ist und nach dem Bersten der Trennschicht infolge Druckeinwirkung des Benutzers der Wirkstoff durch Einwirkung des Aktivierungsmittels hautpermeabel wird.

2. Therapeutisches Pflaster gemäss Anspruch 1, dadurch gekennzeichnet, dass durch chemische Reaktion mit dem Aktivierungsmittel der Wirkstoff hautpermeabel wird.

3. Therapeutisches Pflaster gemäss Anspruch 1, dadurch gekennzeichnet, dass durch Säure-Basen-Reaktion mit dem Aktivierungsmittel der Wirkstoff hautpermeabel wird.

4. Therapeutisches Pflaster gemäss Anspruch 1, dadurch gekennzeichnet, dass durch Reaktion mit einem nicht permeationsfähigen sauren Salz der Wirkstoffbase der Wirkstoff hautpermeabel wird.

5. Therapeutisches Pflaster gemäss Anspruch 1, dadurch gekennzeichnet, dass mit einer gelförmigen Formulierung einer starken Base als Aktivierungsmittel durch Reaktion mit einem pharmazeutisch annehmbaren Säureadditionssalz von Arecolin oder Nicotin die freie Arecolin- oder Nicotinbase hautpermeabel wird.

6. Therapeutisches Pflaster nach einem der Ansprüche 1-5, dadurch gekennzeichnet, dass die undurchlässige Trennschicht (4) eine berstfähige Siegelnaht oder Membranschicht ist.

7. Verfahren zur Herstellung eines therapeutischen Pflasters (1) nach Anspruch 1, dadurch gekennzeichnet,dass man auf der abziehbaren Schutzschicht (8) die Klebschicht (7) und darauf die Membran (6) aufträgt, auf die Membran den Inhalt der beiden Hohlräume (2,3) mit dem Wirkstoff und dem Aktivierungsmittel nebeneinander bringt, über den Inhalt der beiden Hohlräume die Deckschicht (5) legt und diese Deckschicht mit der Membran (6) die beiden Hohlräume (2,3) bildend verschweisst und somit eine undurchlässige Trennschicht (4) bildet, welche durch Druckeinwirkung des Benutzers berstfähig ist, so dass der Wirkstoff durch Einwirkung des Aktivierungsmittels hautpermeabel wird.

## Claims

1. A therapeutic plaster (1) for transdermal or topical application, comprising a reservoir (2, 3) filled with an active ingredient and a further reservoir (2, 3) filled with an activating agent, the two reservoirs being separated by a layer (4) that is impermeable to the activating agent and to the active ingredient, and being bounded on the application side by a membrane (6) having an adhesive layer (7) and a protecting layer (8) applied removably thereto, and being bounded on the side remote from the application side by a backing layer (5), the latter being sealed and impermeable to the active ingredient and/or the activating agent, wherein the filled reservoirs (2, 3) are located next to one another, the separating layer (4) is burstable and, after the separating layer has been burst under pressure applied by the user, the active ingredient becomes capable of permeating the skin as a result of the action of the activating agent.

2. A therapeutic plaster according to claim 1 wherein the active ingredient becomes capable of permeating the skin by chemical reaction with the activating agent.

3. A therapeutic plaster according to claim 1 wherein the active ingredient becomes capable of permeating the skin by acid/base reaction with the activating agent.

4. A therapeutic plaster according to claim 1 wherein the active ingredient becomes capable of permeating the skin by reaction with an acid salt of the active ingredient base that is not capable of permeation.

5. A therapeutic plaster according to claim 1 wherein, with a gel-like formulation of a strong base as activating agent, a free arecoline or nicotine base becomes capable of permeating the skin by reaction with a pharmaceutically acceptable acid addition salt of arecoline or nicotine.

6. A therapeutic plaster according to any one of claims 1 to 5 wherein the impermeable separating layer (4) is a burstable seal or membrane layer.

7. A process for the preparation of a therapeutic plaster (1) according to claim 1, which comprises applying to the removable protecting layer (8) the adhesive layer (7) and applying the membrane (6) thereto, dispensing the contents of the two reservoirs (2, 3) containing the active ingredient and the activating agent side by side on the membrane, placing the backing layer (5) over the contents of the two reservoirs, and heat-sealing the backing layer to the membrane (6) to form the two reservoirs (2, 3) and thus form an impermeable separating layer (4) which is burstable under pressure applied by the user, so that the active ingredient becomes capable of permeating the skin as a result of the action of the activating agent.

## Revendications

1. Emplâtre thérapeutique (1) pour l'application transdermique ou topique, consistant en une cavité (2,3) remplie avec un agent actif et une autre cavité (2,3) remplie avec un agent d'activation, les deux cavités étant séparées par une couche (4) imperméable pour l'agent d'activation et l'agent actif et étant délimitées du côté de l'application par une membrane (6) ayant une couche adhésive (7), ainsi qu'une couche protectrice (8) disposée dessus de manière pelable, et sur la face opposée à la face d'application par une couche de recouvrement (5), cette dernière étant fermée et imperméable vis-à-vis de l'agent actif et/ou de l'agent d'activation, caractérisé en ce que les cavités remplies (2,3) sont juxtaposées, la couche de séparation (4) est brisable et après la rupture de la couche de séparation, sous la pression exercée par l'utilisateur, l'agent actif peut pénétrer à travers la peau sous l'action de l'agent d'activation.

2. Emplâtre thérapeutique selon la revendication 1, caractérisé en ce que l'agent actif est rendu apte à traverser la peau par réaction chimique avec l'agent d'activation.

3. Emplâtre thérapeutique selon la revendication 1, caractérisé en ce que l'agent actif est rendu apte à traverser la peau par réaction acide-base avec l'agent d'activation.

4. Emplâtre thérapeutique selon la revendication 1, caractérisé en ce que l'agent actif est rendu apte à traverser la peau par réaction avec un sel acide inapte à la perméation de la base de l'agent actif.

5. Emplâtre thérapeutique selon la revendication 1, caractérisé en ce que, avec une formulation sous forme de gel d'une base forte en tant qu'agent d'activation, la base arécoline ou nicotine libre est rendue apte à traverser la peau par réaction avec un sel d'addition d'acide pharmaceutiquement acceptable d'arécoline ou de nicotine.

6. Emplâtre thérapeutique selon l'une quelconque des revendications 1-5, caractérisé en ce que la couche de séparation imperméable (4) est un joint de scellement ou une couche membranaire brisable.

7. Procédé pour la production d'un emplâtre thérapeutique (1) selon la revendication 1, caractérisé en ce qu'on applique sur la couche de protection pelable (8) la couche d'adhésif (7) et, par dessus, la membrane (6), on dispose côte à côte sur la membrane le contenu des deux cavités (2,3) comportant l'agent actif et l'agent d'activation, on place sur le contenu des deux cavités la couche de recouvrement (5) et on soude cette couche de recouvrement avec la membrane (6) formant les deux cavités (2,3) et on forme une couche de séparation (4) imperméable, apte à être brisée par l'action de pression de l'utilisateur, si bien que l'agent actif peut pénétrer à travers la peau sous l'action de l'agent d'activation.
